# DEMANDE DE BREVET EUROPEEN

(11) **EP 2 572 658 A1**
(43) Date de publication de la demande: **27.03.2013**
(21) Numéro de dépôt: 12185457.4
(22) Date de dépôt: 21.09.2012
(51) Int. Cl.: A61B 17/17

(54) **Instrumentation chirurgicale pour réaliser une arthrodèse de la cheville, ainsi que kit d'arthrodèse de cheville correspondant**

(30) Priorité: 23.09.2011 FR 1158510
(71) Demandeur: Tornier, 38330 Montbonnot-Saint-Martin (FR)
(72) Inventeur: Lizee, Emmanuel, 38330 Saint Ismier (FR)
(74) Mandataire: Grand, Guillaume

(57) **Abrégé**

Cette instrumentation (1) comporte, d'une part, un dispositif (10) de fixation amovible à l'arrière-pied (F) d'un patient, définissant un axe rectiligne (X12) d'implantation d'un clou d'arthrodèse, et, d'autre part, un dispositif (20) de repérage d'un axe longitudinal (XT) du tibia (T) du patient, adapté pour être assemblé de manière réglable puis verrouillé en position sur le dispositif de fixation lorsque ce dernier est fixé à l'arrière-pied. Afin de respecter davantage la configuration, propre au patient opéré, dans laquelle sa cheville va être bloquée après implantation du clou d'arthrodèse, l'instrumentation selon l'invention prévoit que son dispositif de repérage comporte des moyens (25) de mesure peropératoire, dans un premier plan anatomique prédéterminé, de l'angle entre l'axe d'implantation et l'axe longitudinal du tibia.

## Description

La présente invention concerne une instrumentation chirurgicale pour réaliser une arthrodèse de la cheville d'un patient. L'invention concerne également un kit d'arthrodèse de cheville, comportant une telle instrumentation.

L'invention s'intéresse à la chirurgie orthopédique de la cheville, visant à bloquer cette dernière par un clou d'arthrodèse implanté à travers, à la fois, l'extrémité inférieure du tibia et l'arrière-pied, c'est-à-dire les os du calcanéum et du talus. Plus précisément, l'invention s'intéresse à la mise en place d'un tel clou d'arthrodèse qui est coudé, c'est-à-dire dont les parties tibiale et talo-calcanéenne, qui sont chacune sensiblement rectilignes, sont inclinées l'une par rapport à l'autre, en formant une angulation de coudage de quelques degrés, typiquement de moins de 15°.

Précisément dans ce contexte, WO-A-2010/122034, qui provient du même Demandeur de la présente et sur lequel est basé le préambule de la revendication 1, propose une instrumentation permettant d'implanter un tel clou d'arthrodèse coudé. Cette instrumentation comporte principalement un dispositif de fixation amovible à l'arrière-pied du patient, ainsi qu'un dispositif d'alignement sur un axe longitudinal du tibia, ces deux dispositifs étant conçus pour être assemblés l'un à l'autre de manière réglable puis de manière à être verrouillés en position. Un des principaux intérêts de cette instrumentation est qu'elle permet de contrôler le déplacement du tibia par rapport à l'arrière-pied entre une configuration prédéterminée de perçage de l'arrière-pied et du tibia, pour préparer ainsi ces os à recevoir le clou d'arthrodèse coudé, et une configuration prédéterminée d'arthrodèse, dans laquelle ces os vont être définitivement bloqués sous l'action du clou après son implantation : à cet effet, le dispositif d'alignement précité comprend deux tiges adjacentes que le chirurgien va successivement aligner avec l'axe du tibia, dans un plan anatomique prédéterminé, typiquement un plan frontal au patient, pour passer de manière précise d'une des deux configurations précitées à l'autre, au cours de la chirurgie. Bien entendu, les deux tiges adjacentes forment entre elles, dans le plan anatomique précité, un angle prédéterminé qui est égal à l'angulation de coudage du clou d'ostéosynthèse à implanter.

Bien que l'instrumentation de WO-A-2010/122034 présente ainsi un réel intérêt pour assister le chirurgien qui, jusqu'alors, travaillait exclusivement « à vue », comme bien expliqué dans la partie introductive de WO-A-2010/122034, cette instrumentation n'est pas complètement satisfaisante pour tous les patients, dans le sens où cette instrumentation pré-impose la valeur de l'angle de basculement du tibia par rapport à l'arrière-pied lorsqu'on passe d'une des deux configurations précitées à l'autre. Autrement dit, la configuration d'arthrodèse est en particulier pré-imposée au patient, selon la valeur de l'angulation de coudage du clou d'arthrodèse à implanter.

Le but de la présente invention est d'améliorer l'instrumentation de WO-A-2010/122034 de manière que, tout en étant toujours facile, rapide et pratique à manipuler par le chirurgien, cette instrumentation respecte davantage la configuration, propre au patient opéré, dans laquelle sa cheville va être bloquée en fin d'intervention.

A cet effet, l'invention a pour objet une instrumentation chirurgicale pour réaliser une arthrodèse de la cheville d'un patient, telle que définie à la revendication 1.

Une des idées à la base de l'invention est de chercher à mesurer, au cours de l'intervention d'implantation d'un clou d'arthrodèse coudé, l'angle formé, dans un plan anatomique prédéterminé, typiquement le plan frontal au patient, entre le tibia et l'arrière-pied du patient opéré. De cette façon, alors que le dispositif de fixation appartenant à l'instrumentation conforme à l'invention est fixé à l'arrière-pied du patient et que le dispositif de repérage est verrouillé sur ce dispositif de fixation, ce verrouillage ayant été ajusté en lien avec le plan anatomique précité, le chirurgien peut, en peropératoire, mesurer cet angle entre le tibia et l'arrière-pied lorsque le tibia et l'arrière-pied sont positionnés l'un par rapport à l'autre dans une configuration d'arthrodèse, qui n'est pas pré-imposée mais qui est décidée par le chirurgien au cours de l'intervention en tenant compte, entre autres, de ses constatations anatomiques peropératoires, notamment liées l'environnement tissulaire de l'articulation de la cheville, ainsi qu'en tenant compte d'éventuels souhaits du patient. Sur la base de cette mesure d'angle relative à la configuration d'arthrodèse décidée en peropératoire, le chirurgien peut avantageusement choisir le clou d'arthrodèse le plus approprié, typiquement celui dont l'angulation de coudage est égale ou la plus proche de la valeur d'angle mesurée, parmi plusieurs clous à sa disposition, qui présentent respectivement des valeurs d'angulation de coudage différentes. Puis, après avoir passé le tibia et l'arrière-pied dans une configuration de perçage de ces os, qui est d'ailleurs avantageusement atteinte à l'aide du dispositif de repérage, le chirurgien perce les os de la cheville selon l'axe rectiligne d'implantation défini par le dispositif de fixation. La mise en place du clou d'arthrodèse dans le tunnel réalisé à travers l'arrière-pied et l'extrémité inférieure du tibia permet ensuite de bloquer la cheville dans une configuration identique à ou, en tout cas, très proche de la configuration d'arthrodèse qui a été décidée au cours de la chirurgie. Les performances d'implantation de ce clou d'arthrodèse, ainsi que le confort pour le patient sont alors améliorés.

Des caractéristiques additionnelles avantageuses de l'instrumentation conforme à l'invention, prises isolément ou selon toutes les combinaisons techniquement possibles, sont spécifiées aux revendications 2 à 9.

L'invention a également pour objet un kit d'arthrodèse de cheville, tel que défini à la revendication 10.

L'invention sera mieux comprise à la lecture de la description qui va suivre, donnée uniquement à titre d'exemple et faite en se référant aux dessins sur lesquels :
- les figures 1 et 2 sont des vues en perspective, sous des angles d'observation respectifs différents, d'une instrumentation conforme à l'invention ; et
- les figures 3 à 7 sont des vues en perspective ou en élévation, montrant des étapes successives d'utilisation de l'instrumentation des figures 1 et 2 pour réaliser une arthrodèse de la cheville d'un patient.

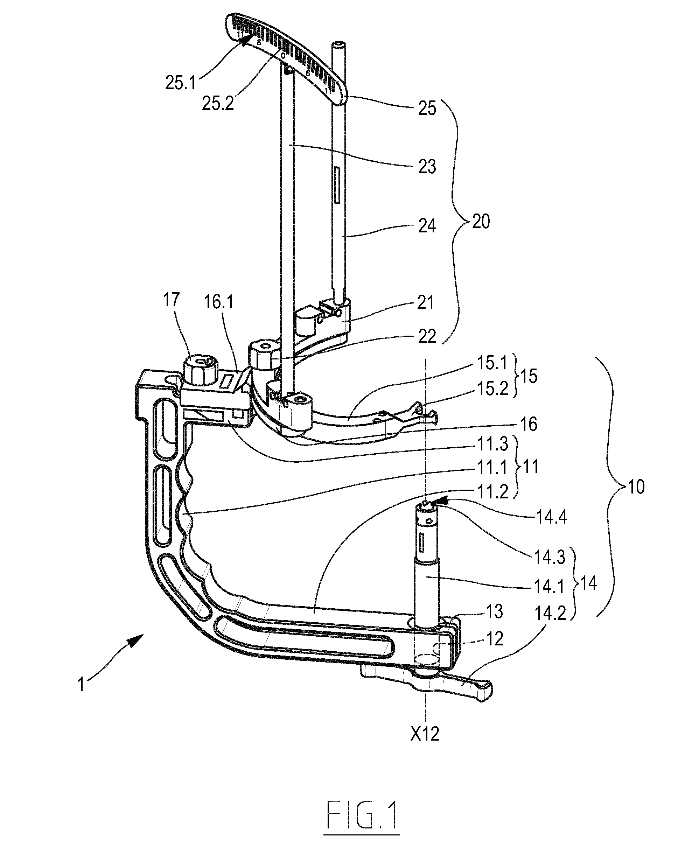

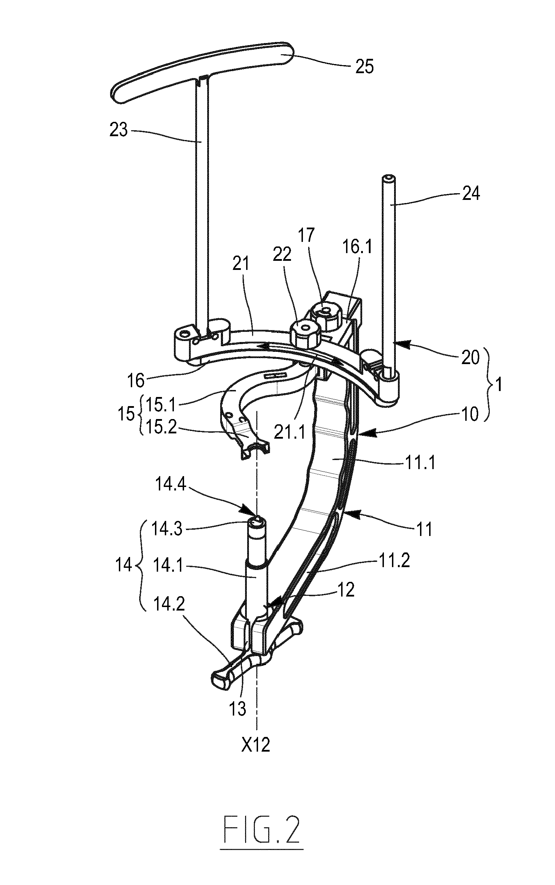

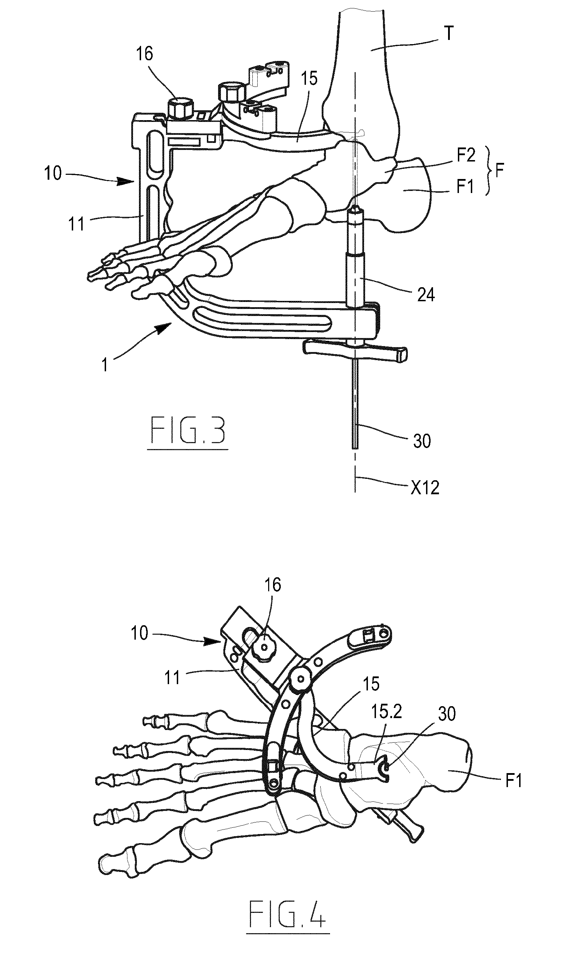

Sur les figures 1 et 2 est représentée une instrumentation chirurgicale 1 qui, comme expliqué plus en détail par la suite, notamment en regard des figures 3 à 7, permet de réaliser une arthrodèse de la cheville d'un patient, plus précisément permet de préparer les os de cette cheville pour que ces derniers reçoivent un clou d'arthrodèse, non représenté, qui est coudé, comme évoqué dans la partie introductive du présent document et comme expliqué plus en détail par la suite.

Comme bien visible sur les figures 1 et 2, l'instrumentation 1 comprend principalement deux dispositifs 10 et 20, qui, comme expliqué par la suite, ont des fonctions respectives distinctes et qui, en service, sont assemblés l'un à l'autre, comme représenté sur les figures 1 et 2. En service, les dispositifs 10 et 20 sont globalement agencés l'un à côté de l'autre, selon une direction qui est sensiblement parallèle à la jambe d'un patient opéré et qui, sur les figures 1 et 2, correspond sensiblement à la direction verticale.

Le dispositif 10, qui occupe la partie basse des figure 1 et 2, comprend un étrier rigide 11 présentant globalement la forme d'un U avec des branches n'ayant pas la même longueur. Ainsi, dans la région de fond de la forme en U précitée, l'étrier 11 inclut une barre principale 11.1, qui s'étend en longueur suivant la direction d'agencement l'un à côté de l'autre des dispositifs 10 et 20. Avantageusement, dans l'exemple de réalisation considéré sur les figures, cette barre 11.1 constitue une poignée de préhension manuelle du dispositif 10, une partie de la surface extérieure de cette barre 11.1 présentant par exemple un relief ondulé dans les creux duquel les doigts de la main du chirurgien peuvent se loger à des fins de préhension.

Aux extrémités longitudinales opposées de la barre 11.1, l'étrier 11 inclut des branches respectives 11.2 et 11.3, qui s'étendent de manière globalement parallèle l'une à l'autre depuis la barre 11.1, conférant ainsi à l'étrier 11 la forme globale de U évoquée plus haut. La branche 11.2, qui est la branche la plus éloignée du dispositif 20 et dont la longueur est supérieure à celle de la branche 11.3, présente, à l'opposé de son extrémité liée à la barre 11.1, une extrémité libre pourvue d'un trou traversant 12 reliant les faces de la branche 11.2, opposées suivant la direction d'agencement l'un à côté de l'autre des dispositifs 10 et 20. Ce trou traversant 12 est centré sur un axe géométrique X12 qui s'étend suivant la direction d'agencement précitée. Ainsi, la barre 11.1 de l'étrier 11 s'étend en longueur de manière sensiblement parallèle à l'axe X12, en étant séparée de cet axe d'une distance valant sensiblement la longueur de la branche 11.2. Pour des raisons qui apparaîtront plus loin, l'extrémité libre de la branche 11.2 délimite également une fente 13, qui relie, de manière sensiblement radiale à l'axe X12, le trou traversant 12 et la surface d'extrémité longitudinale de la branche 11.2, et qui, suivant la direction de l'axe X12, s'étend sur toute l'étendue axiale du trou traversant 12.

Le dispositif 10 comporte également une vis de blocage 14 qui est reçue dans le trou traversant 12, en y étant engagée de manière à pouvoir s'y visser et dévisser par rapport à l'étrier 11. En service, le corps principal de la vis 14 s'étend ainsi suivant l'axe X12, son extrémité axiale tournée à l'opposé du dispositif 20 étant munie d'une tête d'entraînement 14.2, présentant avantageusement une forme de poignée de préhension, tandis que, à son extrémité opposée, la vis 14 est pourvue d'une ou de plusieurs pointes d'accrochage osseux 14.3. Avantageusement, cette ou ces pointes 14.3 sont libres en rotation autour de l'axe X12 par rapport au reste de la vis 14. De plus, pour des raisons qui apparaitront plus loin, la vis 14 est canulée, c'est-à-dire elle délimite intérieurement un alésage traversant 14.4 centré sur l'axe X12.

Le dispositif 10 comporte également une fourchette d'appui osseux 15. Comme bien visible sur la figure 2, cette fourchette 15 comporte un corps principal 15.1 dont une extrémité est fixée, avantageusement de manière amovible, à la branche 11.3 de l'étrier 11, tandis que l'extrémité opposée de ce corps principal 15.1 est conformée en une tête 15.2 en forme de C dont l'évidement est sensiblement centré sur l'axe X12. Avantageusement, la tête 15.2 présente des reliefs d'accrochage osseux, dont l'intérêt apparaitra plus loin lors de la description d'un exemple d'utilisation de l'instrumentation 1.

Le dispositif 10 comporte en outre une barrette 16 conçue pour que le dispositif 20 puisse y être assemblé, comme décrit en détail ci-après. En service, cette barrette 16 est liée fixement au reste du dispositif 10, étant remarqué que, avantageusement, dans le mode de réalisation considéré sur les figures, cette barrette 16 est solidaire d'un capot 16.1, qui recouvre la zone de liaison mécanique entre la branche 11.3 de l'étrier 11 et le corps 15.1 de la fourchette 15 et qui est traversé par une mollette de verrouillage réversible 17.

Quant au dispositif 20, ce dernier comporte une réglette 21 à même d'être montée de manière réglable sur la barrette 16 du dispositif 10. Plus précisément, la position de la réglette 21 par rapport à la barrette 16 est réglable angulairement autour de l'axe X12, comme indiqué schématiquement sur la figure 2 par la flèche à double sens 21.1. En pratique, le montage mobile correspondant de la réglette 21 sur la barrette 16 est réalisé par des moyens mécaniques ad hoc, non visibles sur les figures. A titre d'exemple non limitatif, les moyens précités comportent un système à coulisse, incluant un pion, qui est solidaire d'un des composants parmi la barrette 16 et la réglette 21, et qui est reçu à coulissement dans une rainure délimitée dans l'autre des composants, cette rainure présentant un profil longitudinal en arc de cercle, centré sur l'axe X12.

Le dispositif 20 comporte également une mollette 22 de verrouillage réversible de la position relative entre la barrette 16 et la réglette 21.

Dans l'exemple de réalisation considéré sur les figures, la barrette 16 et la réglette 21 présentent une forme sensiblement d'arc de cercle, centrée sur l'axe X12, avec la mollette 22 agencée dans la portion médiane de la réglette 21. Ces formes en arc de cercle présentent l'avantage d'envelopper la région de la cheville d'un patient, en suivant globalement le pourtour périphérique de cette région. Bien entendu, d'autres géométries de réalisation sont envisageables.

Comme représenté sur les figures 1 et 2, le dispositif 20 comporte en outre deux tiges 23 et 24, qui, dans l'exemple de réalisation considéré sur les figures, sont sensiblement rectilignes. Ces tiges 23 et 24 s'étendent respectivement depuis les extrémités opposées de la réglette 21, de manière sensiblement parallèle à l'axe X12 et en direction opposée au dispositif 10. Pour des raisons qui apparaîtront plus loin, les tiges 23 et 24 sont agencées à 90° l'une de l'autre autour de l'axe X12, ce qui revient à dire que le plan passant par l'axe X12 et par la tige 23 est sensiblement perpendiculaire au plan passant par l'axe X12 et la tige 24.

En service, les tiges 23 et 24 sont liées fixement au reste du dispositif 20. Dans l'exemple de réalisation considéré sur les figures, leurs extrémités tournées vers le dispositif 10 sont fixées, notamment par complémentarité de formes, dans des logements délimités par la réglette 21.

La tige 23 se différencie de la tige 24 par le fait que, à son extrémité tournée à l'opposé du dispositif 10, la tige 23 porte fixement un rapporteur d'angle 25 dont le corps s'étend dans un plan prédéterminé, qui est parallèle au plan passant par l'axe X12 et par la tige 24. Le corps du rapporteur 25 est pourvu de graduations d'angle 25.1 qui sont avantageusement réparties, dans le plan prédéterminé précité, de part et d'autre de la tige 23. Comme bien visible sur la figure 1, ces graduations 25.1 sont quantifiées, ici en degré, à partir d'une graduation de référence 25.2 parmi les graduations 25.1, cette graduation de référence 25.2 étant, dans l'exemple de réalisation considéré sur les figures, associée à la valeur d'angle « 0° ».

D'autres caractéristiques structurelles et fonctionnelles de l'instrumentation 1 vont apparaître ci-après, dans le cadre de la description d'un exemple d'utilisation de cette instrumentation, en regard des figures 3 à 7. Cet exemple d'utilisation est mis en oeuvre au cours d'une intervention chirurgicale visant à réaliser une arthrodèse de la cheville d'un patient, c'est-à-dire visant à bloquer mécaniquement l'un par rapport à l'autre le tibia T et l'arrière-pied F, incluant le calcanéum F1 et le talus F2, du patient, par un clou d'arthrodèse, non visible sur les figures, qui est à implanter à travers le calcanéum F1 et le talus F2 jusqu'à rejoindre l'extrémité inférieure du tibia T. Comme évoqué plus haut, le clou d'arthrodèse précité est coudé, c'est-à-dire que ses parties talo-calcanéenne et tibiale s'étendent en longueur suivant des directions respectives rectilignes, inclinées l'une par rapport à l'autre en formant une angulation de coudage.

Ainsi, sur les figures 3 et 4 qui illustrent une même étape de l'intervention chirurgicale, le dispositif 10 est fixé à l'arrière-pied F. Pour ce faire, le chirurgien se saisit de l'étrier 11, en particulier au niveau de la barre 11.1, et aborde l'articulation de la cheville de manière à, d'une part, placer le trou traversant 12 à l'aplomb de la face plantaire du calcanéum F1 et, d'autre part, à insérer la tête 15.2 de la fourchette 15 dans l'articulation tibio-talienne, notamment jusqu'à positionner le dôme du talus F2 dans l'évidement de la forme en C de cette tête 15.2, comme bien visible sur la figure 4. Moyennant le vissage de la vis 14 dans le trou traversant 12, la ou les pointes 14.3 de cette vis sont appliquées contre la face plantaire du calcanéum F1. L'arrière-pied F se trouve ainsi enserré, suivant l'axe X12, entre cette ou ces pointes 14.3 de la vis 14 et la tête 15.2 de la fourchette 15. Le chirurgien applique de la sorte un effort de serrage suffisant pour assurer la fixation ferme et immobile du dispositif 10 sur l'arrière-pied F, de sorte que le chirurgien peut alors relâcher sa préhension de l'étrier 11. Le dispositif 10 est alors dans la configuration illustrée sur les figures 3 et 4. Bien entendu, on comprend que cette fixation est amovible, le dispositif 10 ayant vocation à être dégagé en fin d'intervention.

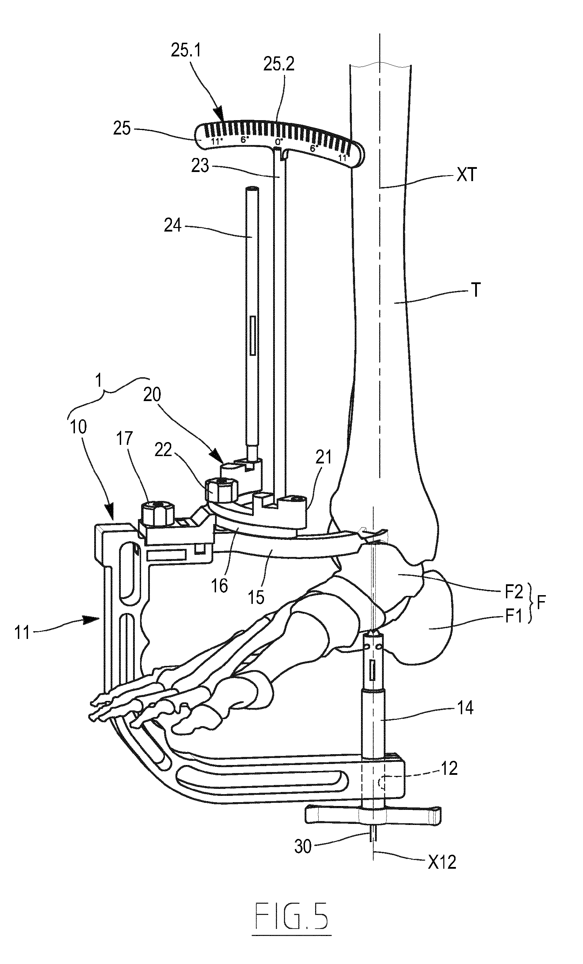

Avant de poursuivre la description de la suite de l'intervention chirurgicale en regard de la figure 5, on notera que, comme bien visible sur la figure 4, le corps principal 15.1 de la fourchette 15 présente l'intérêt de na pas être rectiligne, mais, au contraire, de s'étendre, entre sa tête 15.2 et l'étrier 11, suivant un profil incurvé qui est dimensionné pour contourner des parties anatomiques du patient à conserver. Autrement dit, grâce à ce profil incurvé, la fourchette 15 n'interfère pas avec les parties anatomiques précitées, tout en participant efficacement à l'action de fixation du dispositif 10 sur l'arrière-pied F.

Par ailleurs, à titre d'option particulièrement avantageuse, plutôt que d'utiliser la fourchette 15 montrée sur les figures, le chirurgien peut utiliser une autre fourchette, non représentée sur les figures, qui a la même fonction que la fourchette 15 en ce qui concerne l'action de fixation du dispositif 10 sur l'arrière-pied F, mais qui se distingue de cette fourchette 15 par la forme de son profil incurvé. L'intérêt de mettre ainsi à disposition du chirurgien un jeu d'au moins deux fourchettes à profils différents est lié à la possibilité qu'est ainsi offerte au chirurgien de choisir différentes voies d'abord de la cheville à opérer. A titre d'exemple, la fourchette 15 montrée sur les figures est à utiliser pour une approche antérieure de la cheville, comme bien visible sur la figure 4. Pour approcher la cheville suivant une voie d'abord latérale, une autre fourchette, présentant un profil moins incurvé que celui de la fourchette 15 montrée sur les figures, est préférentiellement utilisée, ce profil moins incurvé étant bien entendu conçu pour, dans le contexte de cette approche latérale de la cheville, contourner des parties anatomiques du patient à préserver. On comprend que le chirurgien est en mesure de changer rapidement et facilement la fourchette qu'il va utiliser au cours de l'intervention chirurgicale. Pour ce faire, il lui suffit d'agir sur la mollette 17 pour, si besoin, dégager la fourchette initialement présente et pour installer ensuite la fourchette qu'il a choisie.

Avant de passer à l'étape de l'intervention illustrée à la figure 5, le chirurgien insère avantageusement une broche de guidage orthopédique 30 dans le calcanéum F1 et dans le talus F2 selon l'axe X12, en la passant par l'alésage central 14.4 de la vis de blocage 14. Comme bien visible sur la figure 4, le chirurgien peut alors vérifier que cette broche 30 émerge du talus F2 au niveau du dôme du talus, c'est-à-dire dans l'évidement de la forme en C de la tête 15.2 de la fourchette 15. Cette broche 30 permet au chirurgien de matérialiser l'axe X12, notamment à des fins de vérification du positionnement de cet axe X12 par rapport à l'arrière-pied F.

A titre de variante de mise en oeuvre, la broche de guidage 30 qui vient d'être évoquée n'est pas mise en place après la fixation amovible du dispositif 10, mais est mise en place avant la mise en place du dispositif 10. Plus précisément, le chirurgien a la possibilité d'insérer la broche 30 dans au moins le calcanéum F1, sans avoir encore manipulé le dispositif 10. Dans ce cas, la mise en place subséquente du dispositif 10 est similaire à ce qui a été décrit plus haut, étant remarqué que la fente 13 permet à la partie de la broche 30, émergeant du calcanéum, d'être passée, latéralement à l'axe X12, depuis l'extérieur jusqu'à l'intérieur du trou traversant 12.

L'intervention chirurgicale se poursuit, avec l'assemblage et le réglage du dispositif 20 sur le dispositif 10. Plus précisément, dans le cadre de l'exemple d'utilisation illustré sur les figures, on note que, dès l'étape illustrée par les figures 3 et 4, une partie du dispositif 20 est pré-assemblée au dispositif 10, à savoir que la réglette 21 et la molette 22 sont pré-assemblées sur la barrette 16. Ceci étant, comme représenté à la figure 5, lors de l'étape chirurgicale subséquente, le reste du dispositif 20 est alors mis en place, à savoir que les tiges 23 et 24 sont rapportées fixement sur la réglette 21. Bien entendu, dans la mesure où la réglette 21 est inutile à la mise en oeuvre de l'étape illustrée par les figures 3 et 4, on comprend que cette réglette 21 peut n'être rapportée sur la barrette 16 qu'au début de l'étape illustrée par la figure 5. De la même façon, dans la mesure où les tiges 23 et 24 sont inutiles à la mise en oeuvre de l'étape illustrée par les figures 3 et 4 sans pour autant empêcher cette mise en oeuvre, on peut envisager, à titre de variante non représentée, que les tiges 23 et 24 soient déjà en place lors de l'étape illustrée par les figures 3 et 4. Quoi qu'il en soit, à partir de l'étape illustrée par la figure 5, le chirurgien dispose de l'assemblage complet des dispositifs 10 et 20 et, comme il ressortira des explications qui vont suivre, le dispositif 20 permet alors au chirurgien de repérer visuellement un axe longitudinal XT du tibia T.

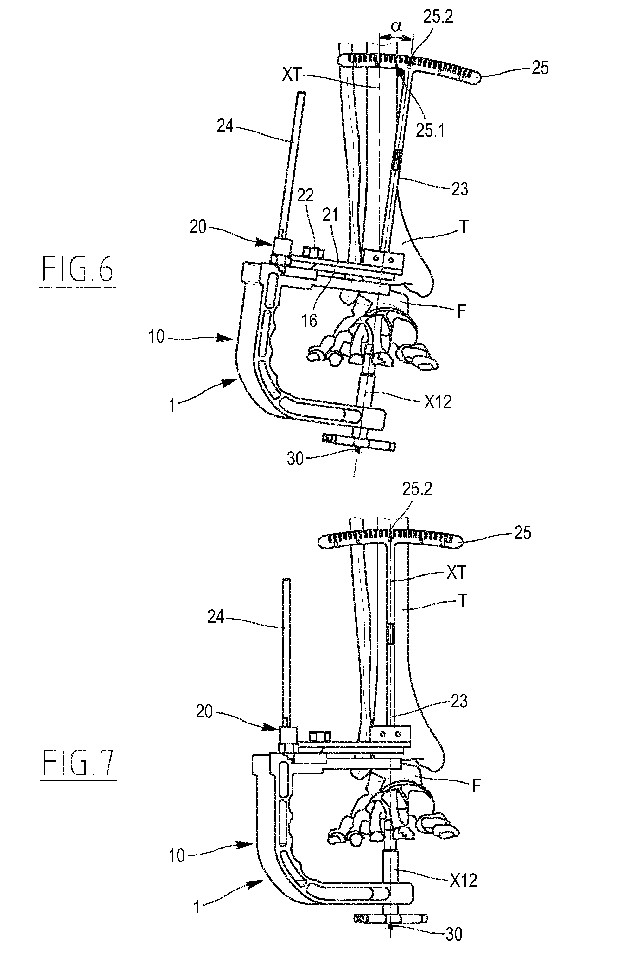

A ce stade, le chirurgien va régler la position de la réglette 21 par rapport à la barrette 16, par rotation de cette réglette 21 autour de l'axe X12. Bien entendu, pour autoriser ce réglage en rotation, la mollette 22 est déverrouillée, puis, une fois que le réglage est finalisé, en tenant compte des explications qui sont développées juste après, cette mollette 22 est verrouillée pour immobiliser en position la réglette 21 par rapport à la barrette 16. L'objectif de ce réglage est de positionner, autour de la cheville, plus précisément autour de l'axe X12, le rapporteur 25, agencé fixement à l'extrémité libre de la tige 23, de sorte que le plan dans lequel s'étend le rapporteur 25 coïncide avec un plan anatomique prédéterminé, par exemple un plan frontal au patient. En fait, le plan anatomique prédéterminé précité est celui dans lequel est caractérisé l'angulation de coudage du clou d'arthrodèse à implanter : en effet, comme on le comprendra au fur et à mesure de la fin de la description de l'intervention chirurgicale, l'instrumentation 1 va fournir au chirurgien des indications positionnelles utiles pour choisir le clou d'arthrodèse parmi plusieurs à sa disposition, ainsi que pour préparer l'implantation du clou d'arthrodèse choisi. Ainsi, à titre d'exemple préférentiel, sur lequel est basée la suite de la présente description, ce plan anatomique prédéterminé est le plan frontal au patient, dans le sens anatomique usuel du terme « frontal ». Avantageusement, ce plan préféré est, plus précisément, le plan frontal du patient, passant par l'axe longitudinal XT du tibia T du patient. On notera que les figures 6 et 7 correspondent à une observation dans ce plan frontal, c'est-à-dire en projection orthogonale dans un plan parallèle au plan frontal du patient. Ceci étant dit, on comprend que, à titre de variantes non représentées, le plan anatomique prédéterminé précité peut être un autre plan que le plan frontal.

En revenant à la description de l'étape illustrée à la figure 5, on comprend que le réglage de la position de la réglette 21 par rapport à la barrette 16 est commandé par le chirurgien de sorte que, par rotation de la réglette autour de l'axe X12, le plan dans lequel s'étend le rapporteur 25 soit positionné dans le plan frontal du patient, par observation directe du chirurgien. Pour aider le chirurgien dans ce réglage, plus précisément pour l'aider dans l'appréciation visuelle de la situation, le chirurgien utilise avantageusement la tige 24 : en observant latéralement le tibia, la mise en place du rapporteur 25 dans le plan frontal revient à positionner la tige 24 dans un plan orthogonal au plan frontal du patient, correspondant au plan sagittal du patient.

L'intervention chirurgicale peut alors se poursuivre, comme illustré par les figures 6 et 7. Ainsi, alors que le dispositif 10 est fixé sur l'arrière-pied F comme décrit plus haut et que le rapporteur 25 a été positionné et verrouillé en position dans le plan frontal du patient, le chirurgien place le pied du patient dans une position qui n'est pas pré-imposée, mais qui est décidée en per-opératoire par le chirurgien, en tenant compte de ces constatations anatomiques per-opératoires, notamment en tenant compte de l'environnement tissulaire de l'articulation de la cheville. La position d'arthrodèse ainsi choisie de manière peropératoire par le chirurgien peut également tenir compte du souhait du patient, notamment lié aux activités régulières de ce patient. Le chirurgien peut par ailleurs tenir compte de données préopératoires, notamment de mesures préopératoires de l'angle sur pied controlatéral, obtenues par scanner, radiographie ou autre. Plus généralement, à ce stade de l'intervention chirurgicale, le chirurgien ne recherche pas une configuration d'arthrodèse préfixée entre l'arrière-pied F et le tibia T : au contraire, le chirurgien ajuste librement la position relative de l'arrière-pied F et du tibia T, jusqu'à aboutir à une configuration d'arthrodèse la plus appropriée au patient. Avantageusement, au cours de ces opérations d'ajustement de la position relative de l'arrière-pied F et du tibia T jusqu'à la configuration d'arthrodèse retenue, le chirurgien utilise avantageusement la tige 24 pour vérifier visuellement que, dans la configuration d'arthrodèse retenue, l'axe longitudinal XT du tibia T s'étend de manière sensiblement parallèle à la tige 24 dans le plan sagittal du patient, c'est-à-dire en projection orthogonale dans ce plan sagittal. L'intérêt de cette vérification apparaitra un peu plus loin.

Une fois que la configuration d'arthrodèse retenue est finalisée, le chirurgien utilise le rapporteur 25 pour mesurer l'angle formé, dans le plan frontal du patient, entre l'axe longitudinal XT du tibia et la tige 23. Dans la mesure où cette tige 23 est parallèle à l'axe X12 défini par l'étrier 11 du dispositif 10, on comprend que le chirurgien mesure, dans le plan frontal du patient, l'angle formé entre l'axe XT du tibia et l'axe X12, comme représenté sur la figure 6. Cela revient à dire qu'il mesure l'angle formé, dans le plan frontal du patient, entre le tibia T et l'arrière-pied F, respectivement repérés par l'axe XT et l'axe X12. Dans l'exemple illustré à la figure 6, cet angle mesuré est noté α et vaut 6° environ, la valeur de cet angle étant avantageusement lue directement sur le rapporteur 25, à l'aide de ses graduations 25.1. A titre optionnel, le chirurgien peut matérialiser l'axe XT du tibia T dans le plan frontal au patient, par exemple soit en dessinant un trait d'axe sur la jambe du patient, soit en utilisant une tige de repérage visuel ou un dispositif de repérage laser, mécaniquement indépendants de l'instrumentation 1.

Ainsi, cet angle mesuré α caractérise la configuration d'arthrodèse retenue, dans le plan frontal du patient : on comprend ainsi que, idéalement, après implantation du clou d'arthrodèse précité, ce clou d'arthrodèse devrait imposer à l'arrière-pied F et au tibia T de retrouver cette configuration décidée en peropératoire. On comprend également au passage l'intérêt de vérifier que, dans la configuration d'arthrodèse retenue, la tige 24 est en alignement sur l'axe XT du tibia dans le plan sagittal du patient car la partie tibiale du clou d'arthrodèse qui va être implantée est destinée à s'étendre selon cet axe XT à l'intérieur du tibia.

Sur la base de la valeur mesurée de l'angle α, le chirurgien décide de la valeur de l'angulation de coudage du clou d'arthrodèse qui va être implanté. Idéalement, le chirurgien choisit alors un clou d'arthrodèse dont l'angulation de coudage à une valeur égale à celle de l'angle mesuré. En pratique, dans la mesure où le chirurgien a à sa disposition un jeu d'au moins deux clous d'arthrodèse, dont les valeurs d'angulation de coudage respectives sont différentes, le chirurgien choisit, parmi les clous d'arthrodèse de ce jeu, celui dont l'angulation de coudage présente la valeur la plus proche de la valeur d'angle mesurée.

L'intervention chirurgicale se poursuit, dans l'objectif de préparer l'arrière-pied F et l'extrémité inférieure du tibia T à recevoir le clou d'arthrodèse qui a été choisi. Ainsi, comme illustré par la figure 7, le chirurgien déplace alors l'arrière-pied F par rapport au tibia T, ce qui revient à dire qu'ils quittent la configuration d'arthrodèse retenue en regard de la figure 6, pour une configuration de perçage, qui est illustrée à la figure 7 et dans laquelle le positionnement relatif de l'arrière-pied F et du tibia T est tel que l'axe X12 et l'axe tibial XT sont coïncidents : dans le plan frontal, cela revient à aligner l'axe XT du tibia sur la tige 23, autrement dit sur la graduation de référence 25.2, c'est-à-dire la graduation « 0° », du rapporteur 25, tandis que, dans le plan sagittal, cela revient à maintenir l'alignement entre la tige 24 et l'axe tibial XT. La broche 30 peut alors être entraînée en direction du tibia T, jusqu'à s'insérer dans l'extrémité inférieure de ce tibia. On comprend qu'on vient de réaliser, dans l'extrémité inférieure du tibia T, un tunnel centré sur l'axe X12, dans le prolongement rectiligne du tunnel précédemment réalisé à travers le calcanéum F1 et le talus F2, de l'arrière-pied F.

A titre de variante de mise en oeuvre, la broche 30 n'est utilisée qu'à ce stade de l'intervention. Autrement dit, le dispositif 10 puis le dispositif 20 sont utilisés comme décrit précédemment mais en l'absence de la tige 30, puis, seulement une fois que le chirurgien est satisfait de la situation, notamment de sa mesure de l'angle α et/ou des configurations d'arthrodèse et de perçage, il insère la broche 30, d'un seul mouvement, à travers le calcanéum F1, puis le talus F2, puis l'extrémité inférieure du tibia T. Cette variante permet notamment au chirurgien de repositionner le dispositif 10 sur l'arrière-pied F, en modifiant les futurs points d'entrée et de sortie de la broche 30, avant d'avoir percé l'arrière-pied.

La suite de l'intervention chirurgicale, qui n'est pas illustrée par des figures, consiste essentiellement à :
- dégager les dispositifs 10 et 20, tout en laissant en place la broche de guidage orthopédique 30, ce qui est d'ailleurs facilité par la possibilité de faire passer la broche 30 dans la fente 13 pour dégager latéralement l'étrier 11 après avoir retiré la vis de blocage 14 ;
- élargir la section transversale des tunnels réalisés à travers l'arrière-pied F et dans l'extrémité inférieure du tibia T, typiquement en utilisant un alésoir engagé autour de la broche de guidage 30 ; et
- mettre en place le clou d'arthrodèse précédemment choisi dans les tunnels élargis délimités dans l'arrière pied F et dans le tibia T, cette implantation étant ainsi réalisée selon l'axe X12.

Lors de la mise en place du clou d'arthrodèse choisi, le chirurgien déplace l'un par rapport à l'autre l'arrière-pied F et le tibia T pour positionner progressivement les parties tibiale et talo-calcanéenne du clou jusque dans leur positionnement final dans le tibia T et l'arrière-pied F, ces derniers se retrouvant alors dans la configuration d'arthrodèse précédemment retenue en regard de la figure 6 ou, à tout le moins, dans une configuration proche de cette configuration d'arthrodèse.

On notera que l'instrumentation 1 décrite jusqu'ici peut avantageusement être utilisée indifféremment sur une cheville gauche et sur une cheville droite d'un patient à opérer. En effet, les figures 3 à 7 illustrent une intervention sur la cheville droite du patient, alors que la cheville gauche peut être traitée de la même façon par l'instrumentation 1, à la différence que :
- parmi les graduations 25.1, on utilise alors les graduations situées du côté de la graduation de référence 25.2, opposé au côté où sont situées les graduations utilisées à la figure 6 ; et
- les tiges 23 et 24 sont alors interchangées, ce qui revient à dire que la tige 23, portant le rapporteur 25, est montée à l'extrémité de la réglette 21, à laquelle est agencée la tige 24 sur les figures 5 à 7, tandis que, dans le même temps, la tige 24 est montée à l'extrémité opposée de la réglette 21, c'est-à-dire à l'extrémité à laquelle est située la tige 23 sur les figures 5 à 7.

Bien entendu, selon que l'instrumentation 1 soit utilisée sur une cheville gauche ou une cheville droite, la fourchette 15 utilisée peut être changée, grâce à sa liaison amovible avec le reste du dispositif 10. En fonction de la voie d'abord choisie, le chirurgien dispose ainsi d'une grande flexibilité instrumentaire.

Par ailleurs, divers aménagements et variantes à l'instrumentation 1, ainsi qu'à sa méthode de mise en oeuvre, sont envisageables. A titre d'exemples :
- le lecteur peut se reporter à WO-A-2010/122034 et s'en inspirer pour modifier ou compléter des aspects techniques de l'instrumentation 1 décrite jusqu'ici ; et/ou
- sur la base des explications en lien avec la tige 24 décrite plus haut, on a compris que cette tige a pour fonction principale de fournir au chirurgien un repérage du plan sagittal ou, plus généralement, du plan perpendiculaire au plan anatomique principal dans lequel l'angle α est mesuré ; par conséquent, d'autres formes de réalisation que celle montrée sur les figures sont envisageables pour cette tige, en particulier en ce qui concerne son agencement au sein du dispositif 20 .

## Revendications

1. Instrumentation chirurgicale (1) pour réaliser une arthrodèse de la cheville d'un patient, comprenant :
- un dispositif (10) de fixation amovible à l'arrière-pied (F) du patient, ce dispositif de fixation définissant un axe rectiligne (X12) d'implantation d'un clou d'arthrodèse, et
- un dispositif (20) de repérage d'un axe longitudinal (XT) du tibia (T) du patient, ce dispositif de repérage étant adapté pour être assemblé de manière réglable puis verrouillé en position sur le dispositif de fixation (10) lorsque ce dispositif de fixation est fixé à l'arrière-pied (F),
**caractérisée en ce que** le dispositif de repérage (20) comporte des moyens (25) de mesure peropératoire, dans un premier plan anatomique prédéterminé, de l'angle (α) entre l'axe d'implantation (X12) et l'axe longitudinal (XT) du tibia (T).

2. Instrumentation suivant la revendication 1, **caractérisée en ce que** les moyens de mesure incluent un rapporteur d'angle (25), qui s'étend dans le premier plan anatomique et qui présente une graduation de référence (25.2) qui, lorsque le dispositif de fixation (10) et le dispositif de repérage (20) sont assemblés l'un à l'autre, est alignée, dans le premier plan anatomique, sur l'axe d'implantation (X12).

3. Instrumentation suivant la revendication 2, **caractérisée en ce que** le rapporteur d'angle (25) présente des graduations (25.1) qui, dans le premier plan anatomique, sont réparties de part et d'autre de la graduation de référence (25.2).

4. Instrumentation suivant l'une des revendications 2 ou 3, **caractérisée en ce que** le dispositif de repérage (20) comporte en outre une tige (23), qui porte à l'une de ses extrémités longitudinales le rapporteur d'angle (25), et qui, lorsque le dispositif de fixation (10) et le dispositif de repérage (20) sont assemblés l'un à l'autre, s'étend en alignement, dans le premier plan anatomique, sur l'axe d'implantation (X12).

5. Instrumentation suivant l'une quelconque des revendications précédentes, **caractérisée en ce que** le dispositif de repérage (20) comporte en outre une tige (24) qui, lorsque le dispositif de fixation (10) et le dispositif de repérage (20) sont assemblés l'un à l'autre, s'étend dans ou parallèlement à un second plan anatomique perpendiculaire au premier plan anatomique, en particulier en étant alignée, dans ce second plan anatomique, sur l'axe d'implantation (X12).

6. Instrumentation suivant l'une quelconque des revendications précédentes, **caractérisée en ce que** le dispositif de repérage (20) comporte en outre une réglette de support (21) qui, en service, porte de manière fixe les moyens de mesure (25), en particulier le rapporteur d'angle (25), ainsi que, le cas échéant, l'une et/ou l'autre desdites tiges (23, 24), cette réglette de support étant adaptée pour être assemblée au dispositif de fixation (10) de manière que le positionnement angulaire, autour de l'axe d'implantation (X12), entre la réglette de support et le dispositif de fixation est réglable puis verrouillable.

7. Instrumentation suivant l'une quelconque des revendications précédentes, **caractérisée en ce que** le dispositif de fixation (10) comporte un étrier (11), qui définit l'axe d'implantation (X12) et qui est pourvu de moyens mécaniques (14, 15) pour enserrer l'arrière-pied (F) suivant la direction de l'axe d'implantation, lesquels moyens mécaniques incluent une vis (14) de blocage sur la face plantaire de l'arrière-pied (F) et une fourchette (15) d'appui sur la face opposée de l'arrière-pied, notamment sur le dôme du talus (F2) de l'arrière-pied.

8. Instrumentation suivant la figure 7, **caractérisée en ce que** l'étrier (11) délimite à la fois un trou traversant (12) de réception de la vis de blocage (14), qui est centré sur l'axe d'implantation (X12), et une fente (13) de passage latéral d'une broche de guidage orthopédique (30), cette fente débouchant de manière sensiblement radiale dans le trou traversant et s'étendant sur toute l'étendue axiale de ce trou traversant.

9. Instrumentation suivant l'une des revendications 7 ou 8, **caractérisée en ce que** le fourchette (15) est liée au reste du dispositif de fixation (10) de manière amovible à des fins d'interchangeabilité entre au moins deux fourchettes différentes selon l'abord chirurgical de la cheville, en particulier entre au moins deux fourchettes qui, dans leur partie (15.1) tournée vers le reste du dispositif de fixation, présentent respectivement des profils incurvés de contournement de parties anatomiques du patient, différents les uns des autres.

10. Kit d'arthrodèse de cheville, comportant :
- un jeu d'au moins deux clous d'arthrodèse coudés, dont les valeurs respectives de leur angulation de coudage sont différentes, et
- des moyens chirurgicaux de sélection et d'implantation d'un des clous d'arthrodèse parmi le jeu, lesquels moyens incluent une instrumentation (1) conforme à l'une quelconque des revendications précédentes.
